# EUROPEAN PATENT APPLICATION

(11) **EP 1 161 945 A2**
(43) Date of publication of application: **12.12.2001**
(21) Application number: 01113850.0
(22) Date of filing: 07.06.2001
(51) Int. Cl.: A61K 31/198, A61K 31/401, A61P 17/02

(54) **Pharmaceutical composition based on proline, glycine and lysine used in the therapy for the healing of tendon lesions and open wounds**

(30) Priority: 07.06.2000 IT MI001257
(71) Applicant: Solartium Establishment, 9490 Vaduz (LI)
(72) Inventor: Dioguardi, Francesco Saverio, 20122 Milan (IT)
(74) Representative: Gervasi, Gemma, Dr.

(57) **Abstract**

Pharmaceutical composition comprising as essential components proline, lysine and glycine, suitable in the therapy for the healing of tendon lesions and open wounds by local injection of an aqueous solution of said composition or application on the wound of powder of the lyophilized product.

## Description

### Summary of the invention

The present invention relates to the preparation of pharmaceutical compositions substantially based on proline, glycine and lysine in injectable form or in form of lyophilized powder product, which can favor fibroblast proliferation with collagen production, in the therapy for the healing of tendon lesions and of wounds, in particular those consequent to surgical operations.

### Prior art

The Italian patent 5,198,465 describes a composition based on proline, glycine and lysine possibly comprising also methionine, cysteine, vitamin C and other components, which can induce or favor the biological synthesis of collagen in those situations in which said synthesis is lacking.

According to this documents of the prior art the aforesaid composition, in which the essential amino acids i.e. proline, glycine, lysine are present in specific weight ratios one to the other, can contain oily or non-oily diluents and excipients, in a suitable form for external topic use.

Or it can be formulated in a suitable form for oral administration.

### Detailed description of the invention

The present invention aims at carrying out a pharmaceutical composition substantially comprising proline, glycine and lysine, which is suitable in the therapy for the healing of tendon lesions and of wounds by local injections or direct application of the lyophilized powdered product.

Beyond the aforesaid three amino acids the composition can contain other amino acids so as to obtain the desired result of promoting the biological synthesis of collagen locally; these various amino acids, which can be present in an amount up to 70% on the total, are particularly useful in case of nutritional deficits.

The amino acids which are used for testing and which are taken into consideration in the present description are those of laevorotatory type, therefore corresponding to the natural products which are to be regarded as the active form: however, also the racemic form can perform the same activity, though on a proportionally lower level.

In general the composition according to the invention is within the following limits:

| | | | |
|---|---|---|---|
| | from 10 | to 50% | by weight |
| glycine | from 10 | to 50% | by weight |
| lysine | from 4 | to 20% | by weight |

Moreover, the following amino acids are particularly useful though not strictly necessary:

| | | | |
|---|---|---|---|
| leucine | from 0 | to 20% | by weight |
| methionine and/or cysteine and/or cystine: as a whole | from 0 | to 20% | by weight |
| valine | from 0 | to 20% | by weight |
| alpha-ketoglutaric acid | from 0 | to 20% | by weight |

Moreover, in many cases the presence of vitamin C in an amount of 10% ÷ 50% on the total is advantageous; vitamin C acts in particular as hydroxylase coenzyme in the catalysis of the biological synthesis of collagen.

The injectable aqueous solution can be advantageously prepared at the time of using it by dissolving the composition according to the invention, previously prepared in lyophilized form, in a biologically compatible aqueous liquid (distilled water, physiological solution or other aqueous solution).

### Product lyophilization

The amino acids are dissolved one after the other in distilled water, the pH value of the clear solution is checked and brought, if necessary, to a value of 6 to 6.4.

The obtained solution, containing about 6% to 8% by weight of total amino acids, is frozen for about 10 hours in a freeze dryer at a temperature of -35°C. The frozen products is placed under a vacuum of at least 80 µHg.

The freezing drying is then carried out with the following parameters:
+20°C fluid in boiler
+22°C circulation pump
- heating timer (5 hours to reach fluid temperature)
- heating with only one resistance, with thermovacuum.

After about 19 hours the product temperature has reached +1.4/+9.6°C. The heating resistance is then switched off and cold fluid is introduced into the boiler, regulating the temperature of the fluid at +42°C and of the circulation pump at +45°C.

The product is kept for 18 hours at the above values under vacuum.

### Product used for in-vitro and in-vivo tests

A product is used whose essential components are:

| | | |
|---|---|---|
| L-proline | 375 parts | ≈ 40.8% by weight (*) |
| L-lysine | 95 p | ≈ 10.3% by weight |
| L-glycine | 450 p | ≈ 48.9% by weight |

| | | |
|---|---|---|
| (*) percentages of single amino acids on the total of amino acids. | | |

This product is identified here and hereinafter as SC-1200.

The composition of amino acids according to the invention has proved highly effective in the therapy for the healing of tendon lesions and of wounds, in particular those consequent to surgical operations.

A topic treatment for tendon lesions is carried out by local injections of a small amount (0.5 to 2 ml) of an injectable aqueous solution containing amounts of 0.05 to 0.3 g of said composition.

The treatment for wounds healing is carried out by injections along wound edges with the same solution in an amount which is proportional to the size of the treated wound.

The healing treatment of wounds can be carried out also through the use of the composition of the invention in form of powdered lyophilized product which is strewn on the surface of the wound.

### In-vitro tests

Cultures of human fibroblasts belonging to the same stock are compared in conditions of standard culture medium (RPMI 1640 + 10% bovine fetal serum, antibiotics) with cultures which have also been treated with various concentrations of SC.1200 corresponding to 1/50 and 1/100 concentrations of a 5% stock solution.

The number of fibroblasts and the production of collagen of types I and III are higher than those which can be detected in the control group to a significant extent both after 48 (P<0.02) and 72 hours (P<0.01).

By calculating the collagen production ratio for every single fibroblast it is possible to obtain a datum which is almost overlapping in the three groups, thus proving that the synthesis induced by SC.1200 is not beyond physiological limits, but it is a time increment followed by a faster duplication of fibroblasts using SC.1200, therefore a higher amount of final product.

### In-vivo tests

The injectable solution used in the tests is in doses of 1-2 ml of physiological solution containing 0.1 g of SC-1200.

In order to evaluate the healing activity of the product SC-1200 we have studied closing times for surgery wounds and the quality of healing in a double-blind study carried out on patients.

The test was carried out on 34 patients suffering from bilateral stenosis of external carotid, who have undergone bilateral re-vascularization.

After the intervention one side at random is treated with the product and the other side with physiological solution (placebo).

The liquids are injected on both edges of the wound with a dose of 2 ml of solution per cm of wound, corresponding for the product to a dose of 100 mg of SC-1200.

The wound is observed every day and healing time corresponds to the one at which all surgical stitches can be removed.

The quality of healing refers to the formation of a well-shaped healing line without keloid-type areas or formation of fistulae and chippings.

The evaluation has not been made by the vascular surgeon but by an aesthetic surgeon.

Table 1 shows healing times in days and the quality of healing in all patients. It can be clearly observed how the administration of SC-1200 can halve the times of recovery for wounded skin with a high statistic significance in favor of the group treated with the product (P<0.01). The wounds close perfectly from the qualitative point of view, no area of hyperplastic tissue has been observed, and only one case of a stitch showing a slight inflammatory process has been detected.

In the group treated with physiological solution two cases with suppurated stitches and one case with a localized healing hyperplasia of about 2-3 cm have been observed.

As a conclusion, local administration by injection in the wounds can accelerate healing of over 3 days (P<0.01), and the formation of cicatricial tissue is wholly normal with no sign of hyperplasia.

**TABLE 1**

| | | | **Healing time (days)** | | **Quality of healing** | |
|---|---|---|---|---|---|---|
| **Patient no.** | **Age (years)** | **Sex (M/F)** | **SC- 1200** | **Phys. Sol.** | **SC-1200** | **Phys. Sol.** |
| 1 | 65 | M | 4 | 8 | Good | Good |
| 2 | 72 | M | 4 | 8 | Good | Good |
| 3 | 67 | M | 4 | 7 | Good | Good |
| 4 | 74 | F | 4 | 8 | Good | Good |
| 5 | 61 | M | 4 | 8 | Good | Good |
| 6 | 58 | M | 5 | 7 | Good | Good |
| 7 | 79 | F | 4 | 8 | Good | Good |
| 8 | 70 | M | 4 | 8 | Good | S.S. |
| 9 | 59 | M | 4 | 8 | Good | Good |
| 10 | 67 | M | 4 | 8 | Good | Good |
| 11 | 59 | F | 4 | 8 | Good | Good |
| 12 | 76 | F | 4 | 7 | Good | Good |
| 13 | 70 | M | 4 | 8 | Good | Good |
| 14 | 66 | M | 5 | 8 | Good | Good |
| 15 | 61 | M | 4 | 8 | Good | Good |
| 16 | 84 | F | 4 | 8 | Good | Good |
| 17 | 75 | M | 4 | 9 | Good | Good |
| 18 | 71 | M | 4 | 7 | Good | Good |
| 19 | 68 | F | 5 | 7 | Good | CLI |
| 20 | 52 | M | 4 | 8 | Good | Good |
| 21 | 76 | M | 4 | 8 | Good | Good |
| 22 | 70 | M | 4 | 8 | Good | Good |
| 23 | 74 | F | 4 | 8 | Good | Good |
| 24 | 69 | M | 4 | 8 | Good | Good |
| 25 | 74 | M | 4 | 7 | Good | Good |
| 26 | 66 | M | 5 | 8 | Good | Good |
| 27 | 72 | M | 4 | 7 | Good | Good |
| 28 | 79 | F | 4 | 8 | S.S | Good |
| 29 | 74 | F | 4 | 8 | Good | Good |
| 30 | 75 | M | 4 | 6 | Good | Good |
| 31 | 74 | M | 5 | 9 | Good | S.S. |
| 32 | 79 | M | 4 | 6 | Good | Good |
| 33 | 78 | F | 4 | 8 | Good | Good |
| 34 | 80 | F | 4 | 6 | Good | Good |
| **Average:** | **70.41** | | **4.15** | **7.67** | | |
| **S.D.:** | **7.3** | | **0.36** | **0.73** | | |

| | | | | | | |
|---|---|---|---|---|---|---|
| S.S.: suppurated stitch | | | | | | |
| S.D.: standard deviation | | | | | | |
| CLI: cicatrix with localized hyperplasia | | | | | | |

In order to evaluate the activity of the product SC-1200 on the ability to recover the continuity of tendon tissue we have chosen as application field partial fractures of the surface flexor tendon of the phalanges of front limbs in horses.

The test is carried out on 30 racehorses, both gallopers and trotters.

The animals show a tendon fracture which can be seen and analyzed in its size by echography. These are therefore anechogenous lesions (tissue discontinuity index) always corresponding to a fiber break with local liquid effusion.

As is known from international literature and from medical practice, said horses should be put to rest for some months, anyway not less than three months to obtain a recovery of collagen fiber bundles.

The horses are randomly divided into two groups and are locally treated under echographic examination, one group with SC-1200 and the other one with physiological solution (placebo) with two injections having a volume of 2 ml, trying to reach the anechogenous area.

The second injection is made 15 days after the previous one. The amount of SC-1200 contained in 2 ml of physiological solution used as carrier for the product is of 100 mg of the amino acid mixture.

The test has been carried out in a double-blind among groups of subjects and the therapeutic evaluation has been made through the wholly objective echographic examination.

The echographic examinations are made before and after the treatments and after 15 days until the 60^{th} day of observation.

The clinical course in the area of the concerned limb is also observed in order to evaluate possible side effects of the treatments.

The treatment is regarded as positive only if a complete curing of the concerned tendon area - by echographic examination - is reached.

14 of the 15 horses treated with SC-1200 show a recovery of tendon fibers in 30 days from the first administration, and in one case after 45 days.

The horses treated with physiological solution show no improvement after 30 days from the first treatment, and only one horse has shown an echographic improvement during the fourth echographic check-up (60 days after the first treatment).

The difference between the two treatments in 30 days of therapy is highly significant with reference to the placebo (P>0.01).

As a conclusion, the administration of two doses near the anechogenous area and of a 2 ml aqueous solution containing 100 mg of amino acid mixture according to the invention can cure tendon lesions within thirty days from the beginning of the treatment, whereas the administration of placebo (physiological solution) does not lead to any improvement.

The composition according to the invention comprising proline, lysine and glycine in the stated ratios, can be advantageously used also as lyophilized product as such, in form of powder to be applied directly on the open wounds. The use of the lyophilized product in powder form has been experimentally proved in 10 clinical cases of surgical wounds after removal of external carotid stenosis. The powder was applied on the wounds starting from the deepest layer. The cicatrization was satisfactory (closing of the wound on average in the fourth day) without occurring of keloid-type areas. In only one case a dehiscence occurred in only one stitch.

In the control group consisting in the same patients subjected to the removal of external counterside carotid stenosis without the treatment of the surgical wounds according to the invention, the closing of the wounds occurred on average at seventh or eighth day and in two cases was remarked subsequently a hyperplasic cicatrix with dehiscence in two stitches of suture.

## Claims

1. Pharmaceutical compositions comprising as essential components proline, lysine and glycine in which the three amino acids are present in a reciprocal amount corresponding to the following percentages by weight: proline 10% to 50%, glycine 10% to 50%, lysine 4% to 20%, suitable in the therapy for the healing of tendon lesions and of open wounds.

2. Composition according to claim 1 containing also other additional amino acids in an amount up to 70% on the total of amino acids.

3. Composition according to claim 2, in which said additional amino acids are chosen among: leucine, methionine, cysteine, cystine, valine, alpha-ketoglutaric acid.

4. Pharmaceutical compositions according to the claim 1 or 2 or 3, in form of injectable aqueous solution.

5. Pharmaceutical composition according to claim 1 in the form of a lyophilized product suitable for the extemporary preparation of an injectable solution.

6. Pharmaceutical compositions according to claim 1 or 2 or 3, in the state of lyophilized product, in form of powder suitable for direct application on the wounds.

7. Composition according to claim 1, **characterized in that** the injectable solution also contains vitamin C.
